# FASCICULE DE BREVET EUROPEEN

(11) **EP 2 079 440 B1**
(45) Date de publication et mention de la délivrance du brevet: **29.04.2015**
(21) Numéro de dépôt: 07858567.6
(22) Date de dépôt: 12.10.2007
(51) Int. Cl.: A61K 9/06, A61K 31/7048, A61K 31/35, A61P 17/10, A61P 17/08, A61P 17/00

(54) **PRODUITS DE SOIN CUTANÉ À DOUBLE COMPARTIMENT COMPRENANT DE L' IVERMECTINE, ET LEURS UTILISATIONS**
ZWEIKAMMER-HAUTPFLEGEPRODUKTE MIT IVERMECTIN UND ANWENDUNGEN DAVON
TWO-COMPARTMENT SKINCARE PRODUCTS COMPRISING IVERMECTIN, AND USES THEREOF

(30) Priorité: 12.10.2006 FR 0654239
(43) Date de publication de la demande: 22.07.2009
(73) Titulaire: Galderma S.A., 6330 Cham (CH)
(72) Inventeur: SEGURA-ORSONI, Sandrine, F-06210 Mandelieu (FR); LOUIS, Fabienne, F-06270 Villeneuve-loubet (FR)
(74) Mandataire: Casalonga
(86) Numéro de dépôt international: PCT/FR2007/052139
(87) Numéro de publication internationale: WO 2008/043974

(56) Documents cités:
- EP-A- 1 668 984
- EP-A2- 1 197 215
- WO-A-03/092583
- WO-A-2004/093886
- US-A1- 2003 064 940
- US-A1- 2004 167 084
- DATABASE WPI Week 200634 Thomson Scientific, London, GB; AN 2006-318140 XP002436631 & CN 1 698 637 A (WANG Y) 23 novembre 2005 (2005-11-23)

## Description

La présente invention se rapporte à un produit de soin cutané contenant une première composition anhydre comprenant au moins un composé de la famille des avermectines, et une seconde composition, comme produit de combinaison pour le traitement des affections dermatologiques.

Les familles des avermectines constitue un groupe de lactones macrocycliques produit par le *bacterium Streptomyces avermitilis* (Reynolds JEF (Ed) (1993) Martindale. The extra pharmacopoeia. 29th Edition. Pharmaceutical Press, Lond*on).* Les avermectines incluent notamment l'ivermectine, l'invermectine, l'avermectine, l'abamectine, la doramectine, l'eprinomectine, l'emamectine et la sélamectine.

L'ivermectine, composé de la famille des avermectines, est un mélange de deux composés appartenant à la classe des avermectines, la 5-0-demethyl-22,23-dihydroavermectine A1a et la 5-0-demethyl-22,23-dihydroavermectine A1b. Ils sont également connus sous le nom de 22,23-dihydroavermectine B1a et 22,23-dihydroavermectine B1b. L'ivermectine contient au moins 80% de 22,23-dihydroavermectine B1a et moins de 20% de 22,23- dihydroavermectine B1b.

Au milieu des années 80, l'ivermectine a été présentée comme médicament anti-parasitaire de large spectre à usage vétérinaire (CAMPBELL, W.C., et al., (1983). Ivermectin: a potent new antiparasitic agent. Science, 221, 823-828.). Elle est efficace contre la plupart des vers intestinaux communs (excepté les ténias), la plupart des acarides, et quelques poux. Elle présente notamment, une affinité importante pour les canaux chlore glutamate-dépendants présents dans les cellules nerveuses et musculaires des invertébrés. Sa fixation sur ces canaux favorise une augmentation de la perméabilité membranaire aux ions chlores, entraînant une hyperpolarisation de la cellule nerveuse ou musculaire. Il en résulte une paralysie neuromusculaire pouvant entraîner la mort de certains parasites. L'ivermectine interagit également avec d'autres canaux chlorure ligand-dépendants tels que ceux faisant intervenir le neuromédiateur GABA (acide gamma-aminobutyrique). L'ivermectine est plus particulièrement un antihelminthique. Elle est déjà décrite chez l'homme dans le traitement de l'onchocercose à *Onchocerca volvulus,* de la strongyloïdose (anguillulose) gastro-intestinale (produit Stromectol®), de la gale sarcoptique humaine (Meinking TL et al., N Engl J Med 1995 Jul 6;333(1):26-30 The treatment of scabies with ivermectin) ainsi que dans le traitement de la microfilarémie diagnostiquée ou suspectée chez les sujets atteints de la filariose lymphatique due à *Wuchereria bancrofti.*

En outre, la demande internationale WO 2004/093886 divulgue l'utilisation de l'ivermectine pour la fabrication d'une composition pharmaceutique topique destinée à l'usage humain. Plus particulièrement, cette demande divulgue l'utilisation de compositions pharmaceutiques topiques destinées à l'usage humain comprenant de l'ivermectine pour le traitement des affections dermatologiques telles que la rosacée, l'acné vulgaire, la dermite séborrhéique, la dermatite périorale, les éruptions acnéiformes, la dermatite acantholytique transitoire, et l'acné *miliaris necrotica.*

Toutefois, la faible compatibilité de l'ivermectine avec de nombreux excipients (N. O. Shaw, M. M. de Villiers and A. P. Lötter, Pharmazie 54 (1999) 5; 372-376 Preformulation stability screening of ivermectin with non-ionic emulsion excipients)*,* ainsi que sa faible solubilité dans l'eau font que les compositions pharmaceutiques contenant de l'ivermectine nécessitent généralement soit l'ajout d'un nombre élevé d'additifs permettant d'obtenir des compositions stables, ce qui a pour effet d'augmenter les risques d'allergies, soit d'être formulées avec des excipients anhydres. Les compositions anhydres rencontrées présentent classiquement l'inconvénient d'un toucher gras et donc d'aspect peu cosmétique, pouvant être responsable d'une diminution de l'observance des patients. En outre, de part la faible stabilité de l'ivermectine dans l'eau, la péremption des compositions aqueuses contenant de l'ivermectine est généralement inférieure à celle de compositions anhydres contenant de l'ivermectine.

Considérant ce qui précède, un problème que se propose de résoudre l'invention est de réaliser un produit de soin cutané stable, comprenant une quantité efficace d'au moins l'ivermectine, dans des formulations adaptées permettant d'éviter la dégradation de l'actif au contact de l'eau ou au contact des matières premières pouvant le dégrader. De plus, le produit devra être choisi de façon à augmenter l'observance des patients.

Par milieu pharmaceutiquement acceptable, on entend un milieu compatible avec la peau, les muqueuses et/ou les phanères.

L'invention a pour objet un produit de soin cutané à double compartiment constitué par un premier compartiment comprenant la première composition anhydre comprenant un principe actif, et un second compartiment comprenant la seconde composition, caractérisé en ce que ledit principe actif est l'ivermectine. L'invention nécessite donc que le principe actif au sein de cette composition anhydre soit soluble et stable. La composition anhydre comprend donc de préférence des constituants simples afin de limiter toute interaction potentiellement gênante du principe actif avec lesdits constituants. Par conséquent, l'accent étant porté sur l'efficacité de la composition anhydre de ce premier compartiment, le second compartiment contient une composition dont l'objectif est avantageusement de conférer un aspect agréable au produit global pour le patient. Selon l'invention, le second compartiment contient une composition aqueuse ou anhydre choisie par l'homme de l'art afin de conférer au produit l'aspect agréable recherché.

Par composition anhydre selon l'invention, on entend une composition comprenant moins de 5% d'eau purifiée en poids par rapport au poids total de la composition.

L'invention a également pour objet l'utilisation dudit produit selon l'invention pour la fabrication d'un médicament destiné au traitement des affections dermatologiques.

En effet, le produit de soin cutané, présenté sous une forme à double compartiment, permet de limiter au maximum les interactions de l'ivermectine, avec les nombreux excipients habituellement contenus dans une composition unique. Les compositions selon l'invention, appliquées simultanément ou successivement, sont ainsi très bien tolérées, précises en terme de quantité d'actif délivré, et d'utilisation pratique. Elles offrent en outre confort et hydratation aux patients.

L'invention sera mieux comprise à la lecture de la description non limitative qui va suivre.

Le produit de soin cutané selon l'invention est composé de préférence de deux compartiments. Ce type de produit à double compartiment, plus communément appelé « Dual Pack » est déjà utilisé dans le domaine de la cosmétique.

A titre d'exemples de modèles de duals packs susceptibles d'être utilisés selon l'invention, on peut citer les duals packs décrits dans les brevets EP0644129, EP0243667 et US5823391. Parmi d'autres modèles de dual packs actuellement commercialisés susceptibles d'être utilisés selon l'invention, on peut citer également, et de manière non limitative :
- les duals packs à sortie unique tel que le modèle duomixer™ commercialisé par la société Maplast™ ou le modèle Symbio dispenser™ commercialisé par la société Airspray™ ;
- les dual packs à sortie double tel que les modèles new duo concept™, duo double actuator™, duo normal™ commercialisés par la société Maplast™.

Selon l'invention, le produit de soin cutané comprend deux compartiments. Le premier compartiment est composé d'une composition anhydre contenant l'ivermectine.

L'ivermectine selon l'invention contient au moins 80% de 22,23-dihydroavermectine B1a et moins de 20% de 22,23-dihydroavermectine B1b.

La composition du premier compartiment selon l'invention peut se présenter sous la forme d'un gel anhydre, d'une émulsion anhydre ou d'une solution anhydre simple, c'est-à-dire comprenant un nombre limité, entre deux et six, de constituants.

La composition du premier compartiment selon l'invention comprend de manière préférentielle de 0,001 à 50% d'ivermectine, en poids par rapport au poids total de la composition. Plus préférentiellement, la composition du premier compartiment comprend de 0,01 à 15% d'ivermectine, en poids par rapport au poids total de la composition.

Selon l'invention, la composition du premier compartiment comprend l'ivermectine, sous une forme solubilisée dans un composé choisi parmi les solvants polaires, les huiles et les solvants amphiphiles, et leurs mélanges.

A titre d'exemples de solvants huileux susceptibles d'entrer dans les compositions du premier compartiment, on peut citer le diisopropyl adipate vendu sous le nom de Crodamol DA par la société Croda, le PPG 15 stéaryl éther vendu sous le nom d'Arlamol E par la société Uniqema, l'octyldodécanol vendu sous le nom d'Eutanol G par la société Cognis, les capric/caprylic triglycérides vendus sous le nom de Miglyol 812 N par la société Sasol, l'alkyl benzoate vendu sous le nom de Tegosoft TN par la société Croda.

A titre d'exemples de solvants polaires susceptibles d'entrer dans les compositions du premier compartiment, on peut citer la N-méthylpyrrolidone vendu sous le nom de Pharmasolve par la société ISP, le diméthylisosorbide vendu sous le nom d'Arlasolve DMI par la société Uniqema, le phénoxyéthanol vendu sous le nom Phenoxetol par la société Clariant, le macrogol 15-hydroxystearate vendu sous le nom de Solutol HS 15 par la société BASF, le propylène glycol vendu par la société Merck, l'alcool benzylique vendu par la société Merck, l'alcool butylique, l'isopropanol et l'éthanol vendus notamment par la société Prolabo, le macrogol 400 vendu sous le nom de Lutrol 400 par la société BASF.

A titre d'exemples de solvants amphiphiles susceptibles d'entrer dans les compositions du premier compartiment, on peut citer le polysorbate 80 vendu sous le nom de Tween 80 V Pharma par la société Uniqema, le poloxamer 124 vendu sous le nom de Synperonic PE/L44 par la société Uniqema, l'alcool oléique vendu sous le nom de HD-Eutanol V PH par la société Cognis, le glycérol triacétate vendu sous le nom de Triacétine par la société Lambert Riviere.

A titre d'exemples d'épaississants lipophiles susceptibles d'entrer dans les compositions du premier compartiment, on peut citer le glycérol distéarate vendu sous le nom de Precirol ATO et le glycérol dibéhénate vendu sous le nom de Compritol 888 par la société Gattefossé, l'alcool cétostéarylique vendu sous le nom de Speziol C18 par la société Cognis, le dioxyde de silicium colloïdal vendu sous le nom d'Aerosil 200 par la société Degussa.

A titre d'exemples de gélifiants susceptibles d'entrer dans les compositions du premier compartiment, on peut citer le gel d'acrylamide vendu sous le nom de Simulgel 600 par la société Seppic, les carbomères vendus sous les noms de Carbopol 980 NF et Carbopol 981 NF, les copolymères d'acrylates/ alkylacrylates vendus sous le nom de Pemulen TR1 et Pemulen TR2 vendus par la société Noveon,ou le glyceryl polymetacrylate and propylene glycol vendu sous le nom de Lubragel CG par la société Guardian, les polysaccharides, les gommes naturelles et les argiles.

La composition du premier compartiment est une solution ou un gel anhydre comprenant un ou des solvants polaires, qui comprend de deux à six constituants dont au moins :
- de 0,1 à 99,99% d'au moins un solvant polaire de l'actif ;
- de 0 à 5% de gélifiant(s) ;
- de 0 à 10% de tensioactif(s)
- de 0,001 à 50% d'ivermectine ;
- de 0 à 3% de conservateur(s).

La composition du second compartiment se présente, selon l'invention, sous la forme d'un véhicule comprenant des excipients cosmétiquement et/ou pharmaceutiquement acceptables, apportant hydratation et confort au patient. Ladite composition peut être aqueuse ou anhydre et se présenter sous la forme d'une émulsion, d'un gel, d'une solution ou d'un onguent, préférentiellement sous la forme d'une émulsion ou d'un gel. Une telle composition comprend ainsi au moins un composé hydratant et/ou émollient ; ces composés peuvent notamment être choisis parmi les huiles (pour leurs propriétés émollientes), ou des composés hydrophiles, incluant l'eau, présents dans la phase aqueuse lorsqu'elle existe.

Le véhicule cosmétiquement et/ou pharmaceutiquement acceptable selon l'invention doit être choisi de telle manière que les propriétés avantageuses attachées intrinsèquement à la présente invention ne soient pas ou substantiellement pas altérées par l'addition envisagée. Le véhicule peut être composé d'un seul excipient tel un solvant, ou d'un mélange d'excipients tels ceux utilisés pour la formulation d'une émulsion. A titre d'exemples d'excipients pouvant être utilisés seuls ou en mélange, on peut citer, l'eau, les solvants, les diluants, tout excipient utilisable pour la formulation d'une émulsion ou d'un gel. Ces excipients sont des composés couramment utilisés dans la formulation de compositions pharmaceutiques. De façon préférentielle, les excipients selon l'invention sont l'eau, les alcools, les polyols, les éthers, les esters, les aldéhydes, les cétones, les acides et alcools gras, les esters gras.

Avantageusement, les compositions selon l'invention sont des émulsions et peuvent comprendre également un ou plusieurs agents tensioactifs dans des concentrations préférentielles allant de 0,1 à 6%.
En outre, les compositions cosmétiques et/ou pharmaceutiques du second compartiment telles que décrites précédemment peuvent en outre comprendre des additifs inertes, ou même pharmacodynamiquement actifs pour ce qui concerne les compositions pharmaceutiques, ou des combinaisons de ces additifs.

Bien entendu, l'homme du métier veillera à choisir le ou les éventuels composés à ajouter à ces compositions et leurs quantités respectives de telle manière que les propriétés avantageuses attachées intrinsèquement à la présente invention ne soient pas ou substantiellement pas altérées par l'addition envisagée.

De préférence, lorsque les compositions du second compartiment se présentent sous forme anhydre, c'est-à-dire comprenant moins de 5% d'eau purifiée en poids du poids total de la composition, il s'agit d'émulsions anhydres ou de gels anhydres.

Selon un premier mode de réalisation avantageux, la composition du second compartiment est une émulsion anhydre comprenant au moins :
- de 8 à 40% d'huile ;
- de 0,5 à 8% d'émulsionnant(s) ayant une balance hydrophile - lipophile (HLB) comprise entre 2 et 7 ;
- de 0 à 5% de coémulsionnant(s) de HLB supérieur à 6 ;
- de 0 à 8% de gélifiant(s) ;
- de 0 à 60% d'humectant(s) ;
- de 0 à 5% d'eau purifiée ; et
- de 0 à 3% de conservateur(s).
- de 4 à 95% d'élastomère de silicone;
- de 2 à 20% d'huile(s); et
- de 0 à 20% d'épaississant(s).

De préférence, lorsque les compositions du second compartiment se présentent sous forme aqueuse, il s'agit d'émulsions aqueuses ou de gels aqueux.

Selon un troisième mode de réalisation avantageux, la composition du second compartiment est une émulsion aqueuse de type gel-crème huile dans eau (H/E), comprenant au moins :
- de 4 à 60% de phase huileuse ;
- de 0,3 à 2% d'émulsionnant(s) polymérique(s) ;
- de 0 à 2% de gélifiant(s) ;
- de 0 à 4% de co-émulsionnant(s) ;
- de 30 à 90% d'eau purifiée ; et
- de 0 à 3% de conservateur(s).

Selon un quatrième mode de réalisation avantageux, la composition du second compartiment est une émulsion aqueuse huile dans eau (H/E), comprenant au moins :
- de 50 à 70% de phase aqueuse ;
- de 0 à 10% d'émulsionnant(s) ;
- de 0 à 4% d'épaississant(s) ;
- de 0,05 à 1% de gélifiant(s) ;
- de 0 à 20% d'humectant(s) ; et
- de 0 à 3% de conservateur(s).

Selon un cinquième mode de réalisation avantageux, la composition du second compartiment est un gel aqueux, comprenant au moins :
- de 50 à 99,7% d'eau purifiée ;
- de 0,3 à 2% de gélifiant(s) ;
- de 0 à 20% d'humectant(s) ; et
- de 0 à 3% de conservateur(s)

Les compositions selon l'invention sont utilisées comme produits pharmaceutiques à usage humain. Plus particulièrement, elles sont utilisées pour le traitement des affections dermatologiques.

Ainsi, l'invention concerne également l'utilisation d'un produit de soin cutané à double compartiment comprenant un premier compartiment composé d'une composition anhydre comprenant au moins l'ivermectine, pour la fabrication d'un médicament destiné au traitement et/ou à la prévention des affections dermatologiques.

Par traitement et/ou prévention des affections dermatologiques, on entend selon la présente invention, le traitement et/ou la prévention de la rosacée, de l'acné vulgaire, de la dermite séborrhéique, de la dermatite périorale, des éruptions acnéiformes, de la dermatite acantholytique transitoire, et de l'acné miliaris necrotica.

Suivant un mode préféré d'utilisation de l'invention, le produit est destiné au traitement de la rosacée.

La présente invention va maintenant être illustrée au moyen des exemples suivants :

### Exemple 1

Etude de la solubilité maximale de l'ivermectine à T = 1 heure à température ambiante dans différents excipients.

| **Excipients** | **Solubilité maximale % (m/m)** |
|---|---|
| N-méthyl 2 pyrrolidone | 58,13 |
| Diméthyl isosorbide | 35,20 |
| Propylène glycol (PG) | 21,83 |
| Phénoxyéthanol | 15,70 |
| Ethanol rectapur | 14,47 |
| Macrogol 15 hydroxystéarate | 10,60 |
| Diisopropyl adipate | 10,40 |
| Polysorbate 80 | 10,31 |
| PEG 400 | 9,14 |
| Poloxamer 124 | 7,50 |
| Triacétine | 7,22 |
| Alcool oléique | 3,48 |
| Capric/caprylic triglycérides | 1,70 |
| Benzoate d'alkyle | 1,60 |

Le tableau ci-dessus présente une liste d'excipients dont le pourcentage maximal de solubilité est supérieur à 1,60. Ces excipients représentent les solvants préférés pouvant être utilisés, seuls ou en mélange, pour la fabrication de compositions simples anhydres comprenant le principe actif ivermectine.

### Exemple 2

Etude comparative de la stabilité chimique de l'ivermectine dans différents excipients :

| Excipients | Teneur à T0 (%) | Stockage à 25 °C pdt 1 mois | Stockage à 40 °C pdt 1 mois |
|---|---|---|---|
| Diisopropyl adipate | 102,5 | 97,19 | 96,70 |
| Alcool benzylique | 98,72 | 99,01 | 98,62 |
| PPG 15-Stéaryl éther | 102,48 | 98,61 | 98,07 |
| Phénoxyéthanol | 99,42 | 97,77 | 98,56 |
| Macrogol 15 hydroxystearate | 98.76 | 95.32 | 71.88 |
| Poloxamer 124 | 98.09 | 98.19 | 92.34 |

Le tableau ci-dessus présente donc les différences de solubilité de l'ivermectine en fonction des excipients. On note notamment une bonne stabilité de l'ivermectine dans le diisopropyl adipate, dans l'alcool benzylique, dans le PPG-15 Stearyl éther et le phénoxyéthanol à température ambiante ou à 40°C. Par contre, l'ivermectine est moins stable dans la macrogol 15 hydroxystéarate et dans le poloxamer, notamment à la température de 40 °C.

### Exemple 3

Produit de soin cutané comprenant un premier compartiment anhydre et un second compartiment aqueux selon l'invention, et son mode de préparation.

### Composition sous forme de gel anhydre du premier compartiment :

| Nom INCI | % formulaire | Phase |
|---|---|---|
| Propylène glycol | 89,40 | A |
| Ethanol rectapur | 5,00 | A |
| Alcool benzylique | 3,00 | A |
| Ivermectine | 2,00 | A |
| Carbomère 980 NF | 0,30 | A |
| Triéthanolamine | 0,30 | A |

### Composition sous forme d'émulsion huile-dans-eau du second compartiment :

| Nom INCI | % formulaire | Phase |
|---|---|---|
| Eau purifiée | Qsp 100% | A' |
| Parahydroxybenzoate de méthyle | 0,25 | A' |
| Glycérol | 8,00 | A' |
| Disodium édetate | 0,10 | A' |
| Allantoïne | 0,20 | A' |
| Carbomère 980 NF | 0,15 | A' |
| Acrylates/C10-30 alkyl acrylate crosspolymer | 0,30 | A' |
| Phénoxyéthanol | 1,00 | A' |
| Parahydroxybenzoate de propyle | 0,10 | B |
| Huile de paraffine | 10,00 | B |
| Sodium hydroxyde (solution à 10%) | Qsp pH 6,3 | C |

### Mode opératoire de la composition du premier compartiment :

La fabrication de la composition du premier compartiment selon l'invention se fait sous lumière inactinique. Les différents constituants de la phase A (voir ci-dessus) sont d'abord pesés, puis le gélifiant est dispersé sous agitation rayneri (pâle défloculeuse) jusqu'à homogénéité. Enfin, la composition est neutralisée.

### Mode opératoire de la composition du second compartiment :

Les différents constituants de la phase B sont d'abord pesés puis chauffés à 65°C. Les constituants de la phase A' sont également pesés, puis chauffés à 65°C puis l'émulsionnant polymérique et le carbomère sont dispersés jusqu'à obtention d'un gel homogène.

On incorpore ensuite la phase B à la phase A' sous agitation rayneri modérée. Enfin, on neutralise le mélange à température ambiante avec la phase C afin d'obtenir un pH de 6,3.

### Exemple 4

Produit de soin cutané comprenant un premier compartiment anhydre et un second compartiment aqueux selon l'invention, et son mode de préparation.

### Composition sous forme de gel anhydre du premier compartiment :

| Nom INCI | % formulaire | Phase |
|---|---|---|
| Diisodopropyl adipate | 89,50 | A |
| glycérol dibéhénate | 5,00 | A |
| Ivermectine | 2,00 | A |

### Composition sous forme d'émulsion huile-dans-eau du second compartiment :

| Nom INCI | % formulaire | Phases |
|---|---|---|
| Eau purifiée | Qsp 100% | A' |
| Méthyle parahydroxybenzoate | 0,15 | A' |
| Glycérol | 10 | A' |
| Disodium édetate | 0,1 | A' |
| Carbomère 980 NF | 0,3 | A' |
| Macrogol 2 stéarylether | 2,5 | B |
| Macrogol 21 stéarylether | 2,5 | B |
| PPG 15 stéarylether | 8,00 | B |
| Propyl parahydroxybenzoate | 0,05 | B |
| Sodium hydroxyde (sol à 10%) | Qsp pH 6,3 | C |

### Mode opératoire de la composition du premier compartiment :

La fabrication de la composition du premier compartiment selon l'invention se fait sous lumière inactinique. Les différents constituants de la phase A sont d'abord pesés, puis le gélifiant est dispersé sous agitation rayneri (pâle défloculeuse) jusqu'à homogénéité.

### Mode opératoire de la composition du second compartiment :

Les différents constituants de la phase B sont d'abord pesés puis chauffés à 65°C. Les constituants de la phase A' sont également pesés, puis chauffés à 65°C, puis le carbomère est dispersé jusqu'à obtention d'un gel homogène.

On incorpore ensuite la phase B à la phase A' sous agitation rayneri modérée. Enfin, on neutralise le mélange à température ambiante avec la phase C afin d'obtenir un pH de 6,3.

### Exemple 5

Produit de soin cutané comprenant un premier et un second compartiment anhydre selon l'invention, et son mode de préparation.

### Composition sous forme de solution anhydre du premier compartiment :

| Nom INCI | % formulaire | Phase |
|---|---|---|
| N méthyle pyrrolidone | 50,00 | A |
| Ethanol rectapur | 20,00 | A |
| Propylène glycol | 29,00 | A |
| Ivermectine | 1,00 | A |

### Composition sous forme de gel anhydre du second compartiment :

| Nom INCI | % formulaire | Phase |
|---|---|---|
| Cyclométhicone/Diméthicone crosspolymer | 92,00 | A' |
| Cyclopentasiloxane | 8,00 | A' |

### Mode opératoire de la composition dupremier compartiment :

La fabrication de la composition du premier compartiment selon l'invention se fait sous lumière inactinique. Les différents constituants de la phase A sont d'abord pesés, puis placés sous agitation rayneri (pâle défloculeuse) jusqu'à homogénéité.

### Mode opératoire de la composition du second compartiment :

Les différents constituants de la phase A' sont d'abord pesés, puis placés sous agitation rayneri (pâle défloculeuse) jusqu'à homogénéité.

### Exemple 6

Produit de soin cutané comprenant un premier compartiment anhydre et un second compartiment aqueux selon l'invention, et son mode de préparation.

### Composition sous forme de solution anhydre du premier compartiment :

| Nom INCI | % formulaire | Phase |
|---|---|---|
| Macrogol 400 | 15,00 | A |
| Propylène glycol | 80,00 | A |
| Polysorbate 80 | 4,50 | A |
| Ivermectine | 0,50 | A |

### Composition sous forme de gel aqueux du second compartiment :

| Nom INCI | % formulaire | Phases |
|---|---|---|
| Eau purifiée | Qsp 100% | A' |
| Glycérol | 20,00 | A' |
| Disodium édetate | 0,1 | A' |
| Carbomère 980 NF | 0,50 | A' |
| Phénoxyéthanol | 1,00 | A' |
| Sodium hydroxyde (sol à 10%) | Qsp pH 6,3 | B |

### Mode opératoire de la composition du premier compartiment :

La fabrication de la composition du premier compartiment selon l'invention se fait sous lumière inactinique. Les différents constituants de la phase A sont d'abord pesés, puis placés sous agitation rayneri (pâle défloculeuse) jusqu'à homogénéité.

### Mode opératoire de la composition du second compartiment :

Les constituants de la phase A' sont d'abord pesés, puis chauffés à 65°C, puis le carbomère est dispersé jusqu'à obtention d'un gel homogène.

On neutralise ensuite le mélange à température ambiante avec la phase B afin d'obtenir un pH de 6,3.

### Exemple 7

Produit de soin cutané comprenant un premier compartiment anhydre et un second compartiment aqueux selon l'invention, et son mode de préparation.

### Composition sous forme de solution anhydre du premier compartiment :

| Nom INCI | % formulaire | Phase |
|---|---|---|
| PPG 15 stearyl éther | 80,00 | A |
| Octyldodécanol | 19,90 | A |
| Ivermectine | 0,10 | A |

### Composition sous forme de gel aqueux du second compartiment :

| Nom INCI | % formulaire | Phases |
|---|---|---|
| Eau purifiée | Qsp 100% | A' |
| Phénoxyéthanol | 1,00 | A' |
| Sorbitol | 10,00 | A' |
| Disodium édetate | 0,10 | A' |
| Acrylamide/sodium Acryloydiméthyltaurate copolymère & isohexadécane & polysorbate 80 | 1,00 | A' |

### Mode opératoire de la composition du premier compartiment :

La fabrication de la composition du premier compartiment selon l'invention se fait sous lumière inactinique. Les différents constituants de la phase A sont d'abord pesés, puis placés sous agitation rayneri (pâle défloculeuse) jusqu'à homogénéité.

### Mode opératoire de la composition du second compartiment :

Les constituants de la phase A' sont d'abord pesés, puis on disperse l'Acrylamide/sodium Acryloydiméthyltaurate copolymère & isohexadécane & polysorbate 80 jusqu'à obtention d'un gel homogène.

### Exemple 8

Produit de soin cutané comprenant un premier compartiment anhydre et un second compartiment anhydre selon l'invention, et son mode de préparation.

### Composition sous forme de solution anhydre du premier compartiment :

| Nom INCI | % formulaire | Phase |
|---|---|---|
| Propylène glycol | 98,50 | A |
| Hydroxyéthylcellulose | 0,50 | A |
| Ivermectine | 1,00 | A |

### Composition sous forme d'émulsion anhydre du second compartiment :

| Nom INCI | % formulaire | Phases |
|---|---|---|
| Lauryl méthicone copolyol | 5,00 | A' |
| Cyclopentasiloxane | 10,00 | A' |
| Huile de paraffine | 15,00 | A' |
| Glycérine | 40,00 | B |
| Glyceryl polyméthacrylate (and) propylene glycol | 8,00 | B |
| Sorbitol | 17,00 | B |
| Eau purifiée | 5,00 | B |

### Mode opératoire de la composition du premier compartiment :

La fabrication de la composition du premier compartiment selon l'invention se fait sous lumière inactinique. Les différents constituants de la phase A sont d'abord pesés, puis placés sous agitation rayneri (pâle défloculeuse) jusqu'à homogénéité.

### Mode opératoire de la composition du second compartiment :

Les constituants de la phase A' et B sont d'abord pesés, puis on incorpore la phase B à la phase A' sous agitation rayneri modérée.

## Revendications

1. Produit de soin cutané à double compartiment, contenant :
(a) un premier compartiment comprenant une première composition anhydre comprenant, dans un milieu pharmaceutiquement acceptable, un principe actif qui est l'ivermectine sous une forme solubilisée dans un composé choisi parmi les solvants polaires, les huiles, les solvants amphiphiles et leurs mélanges, ladite première composition anhydre étant une solution ou un gel anhydre comprenant de deux à six constituants dont au moins (en poids par rapport au poids total de la composition) :
- de 0,1 à 99,99% d'au moins un solvant polaire de l'actif ;
- de 0 à 5% de gélifiant(s) ;
- de 0 à 10% de tensioactif(s)
- de 0,001 à 50% de principe actif ;
- et de 0 à 3% de conservateurs, et
(b) un second compartiment comprenant une seconde composition comprenant des excipients pharmaceutiquement et/ou cosmétiquement acceptables et qui comprend au moins un composé hydratant et/ou émollient,
comme produit de combinaison pour une utilisation simultanée, séparée ou étalée dans le temps dans le traitement d'affections dermatologiques.

2. Produit selon la revendication 1, **caractérisé en ce que** la première composition anhydre comprend entre 0,001% à 50% d'ivermectine en poids par rapport au poids total de la composition.

3. Produit selon la revendication 2, **caractérisé en ce que** la première composition anhydre comprend entre 0,01% à 15% en poids d'ivermectine.

4. Produit selon l'une des revendications précédentes, **caractérisé en ce que** la première composition anhydre se présente sous la forme d'un gel anhydre ou d'une solution anhydre simple.

5. Produit selon l'une des revendications précédentes, **caractérisé en ce que** la seconde composition est anhydre.

6. Produit selon la revendication 5, **caractérisé en ce que** la seconde composition est une émulsion anhydre.

7. Produit selon la revendication 6, **caractérisé en ce que** la seconde composition est une émulsion anhydre comprenant au moins :
- de 8 à 40% d'huile ;
- de 0,5 à 8% d'émulsionnant(s) ayant une balance hydrophile - lipophile ('LB) comprise entre 2 et 7 ;
- de 0 à 5% de coémulsionnant(s) de HLB supérieur à 6;
- de 0 à 8% de gélifiant(s) ;
- de 0 à 60% d'humectant(s) ;
- de 0 à 5% d'eau purifiée ; et
- de 0 à 3% de conservateur(s).

8. Produit selon la revendication 5, **caractérisé en ce que** la seconde composition est un gel anhydre.

9. Produit selon la revendication 8, **caractérisé en ce que** la seconde composition est un gel anhydre comprenant au moins :
- de 4 à 95% d'élastomère de silicone ;
- de 2 à 20% d'huile(s) ; et
- de 0 à 20% d'épaississant(s).

10. Produit selon l'une des revendications 1 à 5, **caractérisé en ce que** la seconde composition est aqueuse.

11. Produit selon la revendication 10, **caractérisé en ce que** la seconde composition est une émulsion aqueuse.

12. Produit selon la revendication 11, **caractérisé en ce que** la seconde composition est un gel-crème comprenant au moins :
- de 4 à 60% de phase huileuse ;
- de 0,3 à 2% d'émulsionnant(s) polymérique(s) ;
- de 0 à 2% de gélifiant(s) ;
- de 0 à 4% de co-émulsionnant(s) ;
- de 30 à 90% d'eau purifiée ; et
- de 0 à 3% de conservateur(s).

13. Produit selon la revendication 11, **caractérisé en ce que** la seconde composition est une émulsion huile dans eau comprenant au moins :
- de 50 à 70% de phase aqueuse ;
- de 0 à 10% d'émulsionnant(s) ;
- de 0 à 4% d'un épaississant(s) ;
- de 0,05 à 1% de gélifiant(s) ;
- de 0 à 20% d'humectant(s) ; et
- de 0 à 3% de conservateur(s).

14. Produit selon la revendication 10, **caractérisé en ce que** la seconde composition est un gel aqueux.

15. Produit selon la revendication 14, **caractérisé en ce que** la seconde composition est un gel aqueux comprenant au moins :
- de 50 à 99,7% d'eau purifiée ;
- de 0,3 à 2% de gélifiant(s) ;
- de 0 à 20% d'humectant(s) ; et
- de 0 à 3% de conservateur(s).

16. Produit selon l'une des revendications précédentes, **caractérisé en ce que** la seconde composition comprend au moins un principe actif.

17. Utilisation du produit de soin cutané selon l'une des revendications 1 à 16 pour la fabrication d'un médicament destiné au traitement et/ou à la prévention des affections dermatologiques.

18. Utilisation selon la revendication 17, **caractérisée en ce que** les affections dermatologiques sont la rosacée, l'acné vulgaire, la dermite séborrhéique, la dermatite périorale, les éruptions acnéiformes, la dermatite acantholytique transitoire, et l'acné *miliaris necrotica.*

19. Utilisation selon la revendication 18, **caractérisée en ce que** l'affection dermatologique est la rosacée.

## Patentansprüche

1. Zweikammerhautpflegeprodukt, das Folgendes enthält:
(a) eine erste Kammer, die eine erste wasserfreie Zusammensetzung umfasst, die in einem pharmazeutisch unbedenklichen Medium einen Wirkstoff umfasst, bei dem es sich um Ivermectin in in einer Verbindung, ausgewählt aus polaren Lösungsmitteln, Ölen, amphiphilen Lösungsmitteln und ihren Mischungen, solubilisierter Form handelt, wobei die erste wasserfreie Zusammensetzung eine wasserfreie Lösung oder ein wasserfreies Gel ist, die/das zwei bis sechs Bestandteile umfasst, von denen mindestens (Gewicht in Bezug auf das Gesamtgewicht der Zusammensetzung):
- 0,1 bis 99,99% mindestens ein polares Lösungsmittel von den Wirkstoff;
- 0 bis 5% Gelbildner;
- 0 bis 10% Tensid(e);
- 0,001 bis 50% Wirkstoff;
- und 0 bis 3% Konservierungsmittel sind, und
(b) eine zweite Kammer, die eine zweite Zusammensetzung umfasst, die pharmazeutisch und/oder kosmetisch unbedenkliche Grundstoffe umfasst und die mindestens eine feuchtigkeitsspendende und/oder zartmachende Verbindung umfasst,
als Kombinationsprodukt für eine gleichzeitige, getrennte oder zeitlich gestaffelte Verwendung bei der Behandlung von dermatologischen Leiden.

2. Produkt nach Anspruch 1, **dadurch gekennzeichnet, dass** die erste wasserfreie Zusammensetzung zwischen 0,001 Gew.-% und 50 Gew.-% Ivermectin in Bezug auf das Gesamtgewicht der Zusammensetzung umfasst.

3. Produkt nach Anspruch 2, **dadurch gekennzeichnet, dass** die erste wasserfreie Zusammensetzung zwischen 0,01 Gew.-% und 15% Gew.-% Ivermectin umfasst.

4. Produkt nach einem der vorhergehenden Ansprüche, **dadurch gekennzeichnet, dass** die erste wasserfreie Zusammensetzung in Form eines wasserfreien Gels oder einer einfachen wasserfreien Lösung vorliegt.

5. Produkt nach einem der vorhergehenden Ansprüche, **dadurch gekennzeichnet, dass** die zweite Zusammensetzung wasserfrei ist.

6. Produkt nach Anspruch 5, **dadurch gekennzeichnet, dass** es sich bei der zweiten Zusammensetzung um eine wasserfreie Emulsion handelt.

7. Produkt nach Anspruch 6, **dadurch gekennzeichnet, dass** es sich bei der zweiten Zusammensetzung um eine wasserfreie Emulsion handelt, die mindestens Folgendes umfasst:
- 8 bis 40% Öl;
- 0,5 bis 8% Emulgator(en) mit einem HLB-Wert zwischen 2 und 7;
- 0 bis 5% Koemulgator(en) mit einem HLB-Wert von über 6;
- 0 bis 8% Gelbildner;
- 0 bis 60% Feuchthaltemittel;
- 0 bis 5% gereinigtes Wasser; und
- 0 bis 3% Konservierungsmittel.

8. Produkt nach Anspruch 5, **dadurch gekennzeichnet, dass** es sich bei der zweiten Zusammensetzung um ein wasserfreies Gel handelt.

9. Produkt nach Anspruch 8, **dadurch gekennzeichnet, dass** es sich bei der zweiten Zusammensetzung um ein wasserfreies Gel handelt, das mindestens Folgendes umfasst:
- 4 bis 95% Silikonelastomer;
- 2 bis 20% Öl(e); und
- 0 bis 20% Verdickungsmittel.

10. Produkt nach einem der Ansprüche 1 bis 5, **dadurch gekennzeichnet, dass** die zweite Zusammensetzung wässrig ist.

11. Produkt nach Anspruch 10, **dadurch gekennzeichnet, dass** die zweite Zusammensetzung eine wässrige Emulsion ist.

12. Produkt nach Anspruch 11, **dadurch gekennzeichnet, dass** die zweite Zusammensetzung eine Gelcreme ist, die mindestens Folgendes umfasst:
- 4 bis 60% Ölphase;
- 0,3 bis 2% polymere(n) Emulgator(en);
- 0 bis 2% Gelbildner;
- 0 bis 4% Koemulgator(en);
- 30 bis 90% gereinigtes Wasser; und
- 0 bis 3% Konservierungsmittel.

13. Produkt nach Anspruch 11, **dadurch gekennzeichnet, dass** die zweite Zusammensetzung eine Öl-in-Wasser-Emulsion ist, die mindestens Folgendes umfasst:
- 50 bis 70% wässrige Phase;
- 0 bis 10% Emulgator(en);
- 0 bis 4% Verdickungsmittel;
- 0,05 bis 1% Gelbildner;
- 0 bis 20% Feuchthaltemittel; und
- 0 bis 3% Konservierungsmittel.

14. Produkt nach Anspruch 10, **dadurch gekennzeichnet, dass** die zweite Zusammensetzung ein wässriges Gel ist.

15. Produkt nach Anspruch 14, **dadurch gekennzeichnet, dass** die zweite Zusammensetzung ein wässriges Gel ist, das mindestens Folgendes umfasst:
- 50 bis 99,7% gereinigtes Wasser;
- 0,3 bis 2% Gelbildner;
- 0 bis 20% Feuchthaltemittel; und
- 0 bis 3% Konservierungsmittel.

16. Produkt nach einem der vorhergehenden Ansprüche, **dadurch gekennzeichnet, dass** die zweite Zusammensetzung mindestens einen Wirkstoff umfasst.

17. Verwendung des Hautpflegeprodukts nach einem der Ansprüche 1 bis 16 zur Herstellung eines Arzneimittels für die Behandlung und/oder Vorbeugung von dermatologischen Leiden.

18. Verwendung nach Anspruch 17, **dadurch gekennzeichnet, dass** es sich bei den dermatologischen Leiden um Rosazea, Acne vulgaris, Dermatitis seborrhoides, Perioraldermatitis, akneähnliche Hautausschläge, vorübergehende akantholytische Dermatitis und Acne miliaris necrotica handelt.

19. Verwendung nach Anspruch 18, **dadurch gekennzeichnet, dass** es sich bei dem dermatologischen Leiden um Rosazea handelt.

## Claims

1. Double compartment skincare product, containing:
(a) a first compartment comprising a first anhydrous composition comprising, in a pharmaceutically acceptable medium, an active ingredient which is ivermectin in a form solubilized in a compound chosen from polar solvents, oils, amphiphilic solvents and mixtures thereof, said first anhydrous composition being an anhydrous solution or gel comprising from two to six constituents among which at least (by weight relative to the total weight of the composition):
- 0.1 to 99.99% of at least one polar solvent of the active agent;
- 0 to 5% of gelling agent(s);
- 0 to 10% of surfactant(s);
- 0.001 to 50% of active ingredient;
- and 0 to 3% of preservatives, and
(b) a second compartment comprising a second composition comprising pharmaceutically and/or cosmetically acceptable excipients and which comprises at least one moisturizing and/or emollient compound,
as a combination product for use in the treatment of dermatological conditions simultaneously, separately or spread out over time.

2. Product according to Claim 1, **characterized in that** the first anhydrous composition comprises between 0.001% and 50% of ivermectin by weight relative to the total weight of the composition.

3. Product according to Claim 2, **characterized in that** the first anhydrous composition comprises between 0.01% and 15% by weight of ivermectin.

4. Product according to one of the preceding claims, **characterized in that** the first anhydrous composition is provided in the form of an anhydrous gel or a simple anhydrous solution.

5. Product according to one of the preceding claims, **characterized in that** the second composition is anhydrous.

6. Product according to Claim 5, **characterized in that** the second composition is an anhydrous emulsion.

7. Product according to Claim 6, **characterized in that** the second composition is an anhydrous emulsion comprising at least:
- 8 to 40% of oil;
- 0.5 to 8% of emulsifier(s) having a hydrophilic-lipophilic balance (HLB) between 2 and 7;
- 0 to 5% of coemulsifier(s) having an HLB greater than 6;
- 0 to 8% of gelling agent(s);
- 0 to 60% of humectant(s);
- 0 to 5% of purified water; and
- 0 to 3% of preservative(s).

8. Product according to Claim 5, **characterized in that** the second composition is an anhydrous gel.

9. Product according to Claim 8, **characterized in that** the second composition is an anhydrous gel comprising at least:
- 4 to 95% of silicone elastomer;
- 2 to 20% of oil(s); and
- 0 to 20% of thickener(s).

10. Product according to one of Claims 1 to 5, **characterized in that** the second composition is aqueous.

11. Product according to Claim 10, **characterized in that** the second composition is an aqueous emulsion.

12. Product according to Claim 11, **characterized in that** the second composition is a gel cream comprising at least:
- 4 to 60% of oily phase;
- 0.3 to 2% of polymeric emulsifier(s);
- 0 to 2% of gelling agent(s);
- 0 to 4% of coemulsifier(s);
- 30 to 90% of purified water; and
- 0 to 3% of preservative(s).

13. Product according to Claim 11, **characterized in that** the second composition is an oil-in-water emulsion comprising at least:
- 50 to 70% of aqueous phase;
- 0 to 10% of emulsifier(s);
- 0 to 4% of thickener(s);
- 0.05 to 1% of gelling agent(s);
- 0 to 20% of humectant(s); and
- 0 to 3% of preservative(s).

14. Product according to Claim 10, **characterized in that** the second composition is an aqueous gel.

15. Product according to Claim 14, **characterized in that** the second composition is an aqueous gel comprising at least:
- 50 to 99.7% of purified water;
- 0.3 to 2% of gelling agent(s);
- 0 to 20% of humectant(s); and
- 0 to 3% of preservative(s).

16. Product according to one of the preceding claims, **characterized in that** the second composition comprises at least one active ingredient.

17. Use of the skincare product according to one of Claims 1 to 16, for the manufacture of a medicament intended for the treatment and/or prevention of dermatological conditions.

18. Use according to Claim 17, **characterized in that** the dermatological conditions are rosacea, acne vulgaris, seborrhoeic dermatitis, perioral dermatitis, acneform rash, transient acantholytic dermatitis and acne *miliaris necrotica.*

19. Use according to Claim 18, **characterized in that** the dermatological condition is rosacea.
